# EUROPÄISCHE PATENTSCHRIFT

(11) **EP 3 600 177 B1**
(45) Veröffentlichungstag und Bekanntmachung des Hinweises auf die Patenterteilung: **29.07.2020**
(21) Anmeldenummer: 17730650.3
(22) Anmeldetag: 09.05.2017
(51) Int. Cl.: A61F 5/042, A61F 5/048, A61F 5/058

(54) **STRECKVORRICHTUNG ZUR VERSORGUNG VON KNOCHENBRÜCHEN**
STRETCHING DEVICE FOR TREATING BONE FRACTURES
DISPOSITIF D'ÉTIRAGE POUR TRAITER DES FRACTURES OSSEUSES

(43) Veröffentlichungstag der Anmeldung: 05.02.2020
(73) Patentinhaber: Kartsana Medical GmbH, 73117 Wangen (DE)
(72) Erfinder: HEHENWARTER, Tobias, 80639 München (DE); HECKELSMÜLLER, Stefan, 80331 München (DE)
(74) Vertreter: Jakelski & Althoff Patentanwälte PartG mbB
(86) Internationale Anmeldenummer: PCT/DE2017/100394
(87) Internationale Veröffentlichungsnummer: WO 2018/206019

(56) Entgegenhaltungen:
- DE-C- 522 975
- US-A- 2 172 484
- US-A- 4 174 709

## Beschreibung

Die vorliegende Erfindung betrifft eine Streckvorrichtung zur Versorgung von Knochenbrüchen. Diese kann insbesondere zur Erstversorgung von Beinbrüchen verwendet werden.

### Stand der Technik

Streckvorrichtungen zur Behandlung von Knochenbrüchen in Arztpraxen sind seit langem bekannt. So beschreibt beispielsweise die DE 522 975 bereits eine Streckvorrichtung mit ein Bein haltenden, zwischen gelenkig verbundene, beliebig einstellbare Schienen festgelegten Manschetten und Hülsen und durch Handkurbelantrieb betätigten Seilzügen. Federnde Spannglieder am Hauptseil werden je nach aufzuwendender Kraft mehr oder wenig gespannt. Eine solche Spannvorrichtung ermöglicht es ein Bein zu strecken, wenn sich beispielsweise die Knochenhälften eines gebrochenen Oberschenkelhalses gegeneinander verschoben haben. Die Bruchstelle kann bei gestrecktem Bein eingerichtet werden und die Streckspannung alsdann wieder vermindert werden, so dass die Knochenteile in die normale Lage kommen. Derartige Streckvorrichtungen können allerdings nur stationär in einer Arztpraxis oder einem Krankenhaus zum Einsatz kommen und sind daher für die Erstversorgung von Knochenbrüchen ungeeignet.

Bei einem Oberschenkelhalsbruch kommt es zu einer unwillkürlichen Kontraktion der Beinmuskulatur, die so lange anhält bis das Bein gestreckt wird. Wenn die Kontraktionen über einen längeren Zeitraum anhalten, kommt es zu einem krampfartigen Zucken der Muskeln, das vom Verletzten nicht kontrolliert werden kann. Dies verursacht Komplikationen in der Behandlung des gebrochenen Oberschenkelhalses, insbesondere dann, wenn die Knochenhälften gegeneinander verschoben sind oder sogar in das umgebende Muskelgewebe stechen. Derartige Krämpfe können für den Verletzten lebensgefährlich werden, da durch sie große Arterien und andere Blutgefäße in der Nähe des Oberschenkelhalses verletzt werden können. Dies kann rapiden Blutverlust verursachen, weicher innerhalb weniger Minuten zum Tode führen kann. Es ist deshalb unbedingt erforderlich, das gebrochene Bein noch am Unfallort zu strecken oder anderweitig zu fixieren bis eine weitere Versorgung des Knochenbruchs in einem Krankenhaus erfolgen kann.

Eine Möglichkeit das Bein zu fixieren besteht darin, den Patienten in einer sogenannten Vakuummatraze zu immobilisieren. Vakuummatrazen werden in einem Krankenwagen verwendet, um Patienten mit multiplen Traumata bis zum Eintreffen in einem Krankenhaus zu immobilisieren. Wenn mit dem Krankenwagen nicht unmittelbar an den Unfallort herangefahren werden kann, ist es allerdings zunächst erforderlich, den noch nicht immobilisierten Patienten zum Krankenwagen zu transportieren. Innerhalb von Gebäuden muss er hierzu für einen Transport über Treppen häufig in eine geneigte Position gebracht werden oder in einem Aufzug sogar aufrecht transportiert werden. Bei diesem Transport kann es zu einer Verletzung von Blutgefäßen kommen. Da der Transport für den Patienten außerdem mit erheblichen Schmerzen verbunden ist, ist es oftmals notwendig, ihm starke Schmerzmittel wie beispielsweise Morphine zu verabreichen, bevor überhaupt mit dem Transport begonnen werden kann. Auch beim weiteren Transport in der Vakuummatraze erleidet der Patient starke Schmerzen, da das gebrochene Bein dort zwar immobilisiert, jedoch noch nicht gestreckt wird. Auch dem kann nur durch die Verabreichung starker Schmerzmittel begegnet werden. Um ein gebrochenes Bein bereits am Unfallort strecken zu können, wurden tragbare Streckvorrichtungen entwickelt. Die US 7,641,624 B2 beschreibt beispielsweise eine Streckvorrichtung mit einer an einem Bein anschnallbaren Schiene, die aus mehreren Segmenten besteht. Die Segmente sind hohl und durch eine in diesen verlaufende Schnur miteinander verbunden. Durch Drehen einer Spindel einer Spannvorrichtung, die unterhalb des Fußes des Patienten angebracht wird, kann das Bein gestreckt werden. Dafür muss die Schiene sich weit über den Fuß des Patienten hinaus erstrecken. Der Einsatz einer solchen Streckvorrichtung ist zeitaufwendig, da sie am Unfallort erst aus mehreren Teilen zusammengebaut werden muss. Zeit steht aber im Umgang mit schwerverletzten Patienten nur begrenzt zur Verfügung. Außerdem weist diese Vorrichtung viele offenliegende mechanische Teile auf. Bei der Bergung von Unfallopfern in der freien Natur, die beispielsweise beim Klettern im Gebirge abgestürzt sind, kann sich eine solche Vorrichtung als unzuverlässig erweisen. Durch die über den Fuß des Patienten hinausstehenden Teile wird dessen Abtransport vom Unfallort zudem erschwert. Das Patent-Dokument US 4 174 709 A offenbart eine Streckvorrichtung zur Versorgung von Knochenbrüchen, aufweisend eine Teleskopschiene, welche eine äußere Schiene und eine in diese einschiebbare innere Schiene aufweist, wobei die innere Schiene ein entlang ihrer Längsachse verlaufendes Gewinde aufweist.

Es ist Aufgabe der vorliegenden Erfindung eine Streckvorrichtung zur Versorgung von Knochenbrüchen bereitzustellen, welche transportabel ist und von Rettungssanitätern leicht an verschiedenste Unfallorte mitgeführt werden kann. Sie sollte robust ausgeführt sein, so dass sie auch unter widrigen Bedingungen eingesetzt werden kann und zudem den Abtransport eines Patienten von einem Unfallort nicht behindert.

### Offenbarung der Erfindung

Diese Aufgabe wird durch eine Streckvorrichtung zur Versorgung von Knochenbrüchen gelöst, welche eine Teleskopschiene aufweist. Die Teleskopschiene weist eine äußere Schiene und eine in die äußere Schiene einschiebbare innere Schiene auf. Die innere Schiene verfügt über eine entlang ihrer Längsachse verlaufende Verzahnung. Ein Drehknopf ist an der äußeren Schiene angeordnet. Dieser kann zwischen einer ersten Position und einer zweiten Position bewegt werden, indem er von der äußeren Schiene weggezogen wird und indem er in Richtung der äußeren Schiene gedrückt wird. Die Streckvorrichtung weist weiterhin ein Schneckenzahnrad auf, das eingerichtet ist, um durch Drehung des Drehknopfes gedreht zu werden. Es ist so angeordnet, dass es in der ersten Position des Drehknopfes nicht in die Verzahnung der inneren Schiene eingreift. In der zweiten Position des Drehknopfes greift sie hingegen in die Verzahnung ein.

Diese Streckvorrichtung ist sehr platzsparend, da die innere Schiene beim Transport weitgehend in die äußere Schiene eingeschoben werden kann. Durch Verwendung eines leichten und zugleich belastbaren Materials für die Teleskopschiene wie beispielsweise Aluminium kann die Streckvorrichtung außerdem sehr leicht ausgeführt werden. Solange sich der Drehknopf in der ersten Position befindet, kann die innere Schiene frei in der äußeren Schiene bewegt werden, da das Schneckenzahnrad noch nicht in die Verzahnung der inneren Schiene eingreift. Die Teleskopschiene kann also an einem Unfallort schnell auf die erforderliche Länge ausgezogen werden, um sie am Bein eines Patienten anzubringen. Wie weit sie ausgezogen werden muss, hängt von der Beinlänge des Patienten ab. Das anschließende Strecken des gebrochenen Beins sollte millimetergenau erfolgen. Hierzu wird der Drehknopf in die zweite Position gebracht. Sobald nun das Schneckenzahnrad in die Verzahnung der inneren Schiene eingreift, kann bei einem Drehen des Drehknopfes dessen Drehbewegung über das Schneckenzahnrad und die Verzahnung auf die innere Schiene übertragen werden, um diese so zu teleskopieren. Wenn die innere Schiene und die äußere Schiene am Bein des Patienten fixiert sind, wird dieses dadurch gestreckt. Sobald der gewünschte Streckungsgrad erreicht ist, genügt es auf eine weitere Drehung des Drehknopfes zu verzichten, um das Bein in der erreichten Position zu fixieren. Solange sich der Drehknopf in seiner zweiten Position befindet, bewirkt der Eingriff des Schneckenzahnrades in die Verzahnung eine Selbsthemmung, die ein unbeabsichtigtes Verschieben der inneren Schiene gegenüber der äußeren Schiene verhindert.

Die Teleskopschiene muss lediglich an geeigneten Stellen am Oberschenkel und Unterschenkel des Patienten befestigt werden. Sie steht nicht über den Fuß des Patienten hinaus und erschwert somit weder seinen Transport noch seine spätere Positionierung in einer Immobilisierungsvorrichtung. Somit kann sie auch beim Transport in ein Krankenhaus und gegebenenfalls selbst im Krankenhaus am Bein des Patienten belassen werden.

Es ist bevorzugt, dass die äußere Schiene eine Ausnehmung aufweist. Auf der Ausnehmung ist ein Aufnahmeelement angeordnet. Das Aufnahmeelement weist eine Achse auf, auf der das Schneckenzahnrad angeordnet ist und Führungen, in denen die Achse beweglich angeordnet ist. Diese Bewegung kann orthogonal zur äußeren Schiene erfolgen. Das Aufnahmeelement kann das Schneckenzahnrad so umschließen, dass es vor äußeren Einflüssen geschützt ist. Dabei kann es zudem die Ausnehmung so abdecken, dass keine Fremdkörper in die Ausnehmung gelangen können. Wenn die Achse in den Führungen orthogonal von der äußeren Schiene wegbewegt wird, wird die erste Position erreicht, in der das Schneckenzahnrad nicht in die Verzahnung der inneren Schiene eingreift. Wird es hingegen in Führungen auf die äußere Schiene zu bewegt, so stößt das Schneckenzahnrad mit seiner Verzahnung an die Verzahnung der inneren Schiene an, so dass die zweite Position erreicht wird.

Da auf das Aufnahmeelement keine so großen Kräfte wirken wie auf die Teleskopschiene, ist es nicht erforderlich dieses aus einem Metall herzustellen. Stattdessen kann es beispielsweise aus einem Kunststoff bestehen, und an die Teleskopschiene angeschraubt werden.

Auf der Achse ist vorzugsweise neben dem Schneckenzahnrad auch noch ein Kegelzahnrad angeordnet. Dieses dient der Kraftübertragung zwischen dem Schneckenzahnrad und dem Drehknopf. Hierzu weist der Drehknopf vorzugsweise einen innenliegenden Zahnkranz auf, welcher mit dem Kegelzahnrad eingreift. Das Schneckenzahnrad und das Kegelzahnrad werden dabei vollständig vom Drehknopf und dem Aufnahmeelement umschlossen.

Um zu erreichen, dass ein Drücken oder Herausziehen des Drehknopfes das Schneckenzahnrad zwischen der ersten Position und der zweiten Position hin- und herbewegt, ist es bevorzugt, dass die Achse in den Drehknopf eingreift. Wenn der Drehknopf orthogonal zur Teleskopschiene bewegt wird, wird diese Bewegung somit über die Achse auf das Schneckenzahnrad übertragen. Indem die Achse in den Führungen des Aufnahmeelements geführt ist, ist der Drehknopf auf diese Weise zudem mit dem Aufnahmeelement verbunden und kann nicht von diesem gelöst werden.

Ein maximales Ausfahren der inneren schiene aus der äußeren Schiene kann insbesondere dadurch ermöglicht werden, dass der Drehknopf an dem der inneren Schiene zugewandten Ende der äußeren Schiene angeordnet ist.

Zur Befestigung der Teleskopschiene am Bein eines Patienten weist diese insbesondere Gurtbefestigungselemente auf. Unter Gurtbefestigungselementen werden Elemente verstanden, an denen ein Gurt so befestigt werden kann, dass er eine Gliedmaße oder den Körper eines Patienten umschließen kann. Zumindest eines der Gurtbefestigungselemente ist mit der äußeren Schiene verbunden und zumindest eines der Gurtbefestigungselemente ist mit der inneren Schiene verbunden, damit eine Teleskopierung der Teleskopschiene in eine Streckung des Beines umgesetzt werden kann. Der Drehknopf ist dabei insbesondere auf einer von einem Gurtbefestigungselement abgewandten Seite der Teleskopschiene angeordnet. Es ist erforderlich, den Drehknopf so anzuordnen, dass er nicht dem Bein eines Patienten zugewandt wird, um ihn nach Anbringen der Streckvorrichtung am Bein eines Patienten bedienen zu können. Wird er dabei zugleich so angeordnet, dass er einem der Gurtbefestigungselemente gegenüberliegt, wird eine besonders günstige Krafteinwirkung auf das Bein erreicht.

Es ist weiterhin bevorzugt, dass die Teleskopschiene ein Beckengurtbefestigungselement aufweist, das mit der äußeren Schiene verbunden ist. Dieses sorgt dafür, dass das Bein des Patienten nicht nur gestreckt, sondern zusätzlich immobilisiert wird. Ein mit dem Beckengurtbefestigungselement verbundener Beckengurt kann neben der Fixierung der Streckvorrichtung gegebenenfalls auch zur Versorgung eines Beckenbruches dienen. Es ist daher bevorzugt, dass das Beckengurtbefestigungselement trennbar von der Teleskopschiene ausgeführt ist, so dass ein für den konkreten Anwendungsfall am besten geeignetes Beckengurtbefestigungselement mit der Teleskopschiene kombiniert werden kann.

Um ein maximales Einfahren der inneren Schiene in die äußere Schiene zu ermöglichen, weist die innere Schiene vorzugsweise nur ein einziges Gurtbefestigungselement auf, das an ihrem der äußeren Schiene abgewandten Ende angeordnet ist. Dieses ist insbesondere als Fußgurtbefestigungseiement ausgeführt.

Je nachdem, ob die Streckvorrichtung an einem linken Bein oder an einem rechten Bein eines Patienten angebracht werden soll, ist eine unterschiedliche Anordnung der Gurtbefestigungselemente vorteilhaft. Die Gurtbefestigungselemente sind deshalb vorzugsweises so mit der Teleskopschiene verbunden, dass sie an dieser um 180° gedreht werden können. Eine solche drehbare Verbindung kann in einer Ausführungsform der Streckvorrichtung erfolgen, indem die Gurtbefestigungselemente jeweils an nur einem einzigen Verbindungspunkt mit der Teleskopschiene verbunden sind. Diese Verbindung kann insbesondere so erfolgen, wie es in der WO 2016/089679 A1 beschrieben wird, die vollständig zum Teil dieser Offenbarung gemacht wird. In einer anderen Ausführungsform sind in den Gurtbefestigungseiementen jeweils zwei Magnete eingebracht. Diese Magnete ziehen in zwei Positionen einen Stahleinleger in der Teleskopschiene an. Dadurch können sie zwischen zwei Rastpositionen gedreht werden.

Wenn das Beckengurtbefestigungselement von der Teleskopschiene trennbar ausgeführt ist, kann es vorzugsweise so an dieser angebracht werden, dass es sowohl in einer für die Befestigung der Teleskopschiene am linken Bein als auch am rechten Bein geeigneten Position mit der Teleskopschiene verbunden werden kann.

### Kurze Beschreibung der Zeichnungen

Ein Ausführungsbeispiel der Erfindung ist in den Zeichnungen dargestellt und wird in der nachfolgenden Beschreibung näher erläutert.
- Fig. 1: zeigt eine isometrische Darstellung einer Streckvorrichtung gemäß einem Ausführungsbeispiel der Erfindung.
- Fig. 2: zeigt in einer isometrischen Darstellung, wie eine Streckvorrichtung gemäß einem Ausführungsbeispiel der Erfindung an einem Bein eines Patienten angebracht werden kann.
- Fig. 3: zeigt die Anordnung eines Drehknopfes und eines Aufnahmeelements an einer Teleskopschiene in isometrischer Darstellung in einem Ausführungsbeispiel der erfindungsgemäßen Streckvorrichtung.
- Fig. 4: zeigt eine Explosionsdarstellung des Drehknopfes und des Aufnahmeelements an der Teleskopschiene gemäß Fig. 3.
- Fig. 5: zeigt eine Schnittdarstellung eines Ausschnitts einer Streckvorrichtung gemäß einem Ausführungsbeispiel der Erfindung, wobei sich der Drehknopf in einer ersten Position befindet.
- Fig. 6: zeigt eine Schnittdarstellung der Streckvorrichtung gemäß Fig. 5, wobei sich der Drehknopf in einer zweiten Position befindet.

### Ausführungsbeispiele der Erfindung

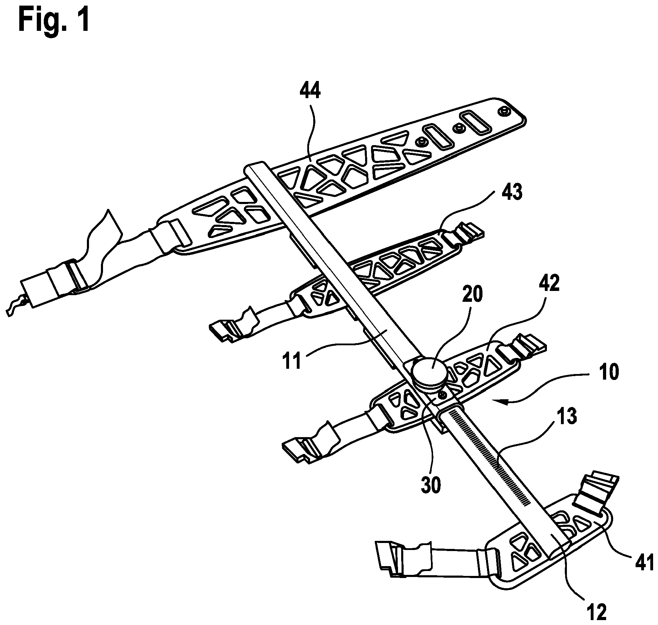

In Fig. 1 ist eine Streckvorrichtung zur Versorgung von Knochenbrüchen gemäß einem Ausführungsbeispiel der Erfindung dargestellt. Diese weist eine Teleskopschiene 10 auf, die vorliegend aus Aluminium besteht. Die Teleskopschiene 10 weist eine äußere Schiene 11 und eine teilweise in die äußere Schiene 11 eingeschobene innere Schiene 12 auf. An dem der inneren Schiene 12 zugewandten Ende der äußeren Schiene 11 ist ein Drehknopf 20 mittels eines Aufnahmeelements 30 mit der äußeren Schiene 11 verbunden. Die innere Schiene 12 weist entlang ihrer Längsachse eine Verzahnung 13 auf ihrer dem Drehknopf 20 zugewandten Seite auf. An dem von der äußeren Schiene 11 abgewandten Ende der inneren Schiene 12 ist ein Gurtbefestigungselement 41 in Form eines Fußgurtbefestigungselements angeordnet. Weitere Gurtbefestigungselemente 42, 43 sind an der äußeren Schiene angeordnet und als Beingurtbefestigungselemente ausgeführt. Dabei ist eines dieser Gurtbefestigungselemente 42 auf der dem Drehknopf 20 gegenüberliegenden Seite der äußeren Schiene 11 angeordnet. An dem von der inneren Schiene 12 abgewandten Ende der äußeren Schiene 11 ist ein Gurtbefestigungselement 44 angeordnet, das als Hüftgurtbefestigungselement ausgeführt ist. Alle Gurtbefestigungselemente 41, 42, 43, 44 sind auf der dem Drehknopf 20 abgewandten Seite der Teleskopschiene 10 angebracht.

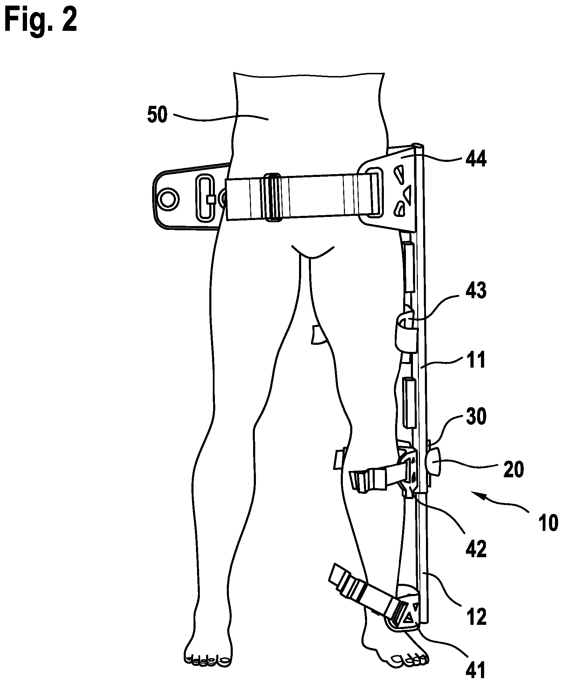

Fig. 2 zeigt wie die Streckvorrichtung mittels der Gurtbefestigungselemente 41, 42, 43, 44 am Becken und an einem Bein eines Patienten 50 befestigt werden kann. Das Beckengurtbefestigungselement 44 ist von der Teleskopschiene 10 trennbar ausgeführt. Es kann zunächst am Becken des Patienten 50 fixiert werden und anschließend mit der Teleskopschiene 10 verbunden werden. Diese wird dann entsprechend der Beinlänge des Patienten ausgefahren und mittels der übrigen Gurtbefestigungselemente 41, 42, 43 am Bein des Patienten befestigt. Anschließend kann ein Strecken des Beines erfolgen, um beispielsweise einen Oberschenkelhalsbruch zu versorgen.

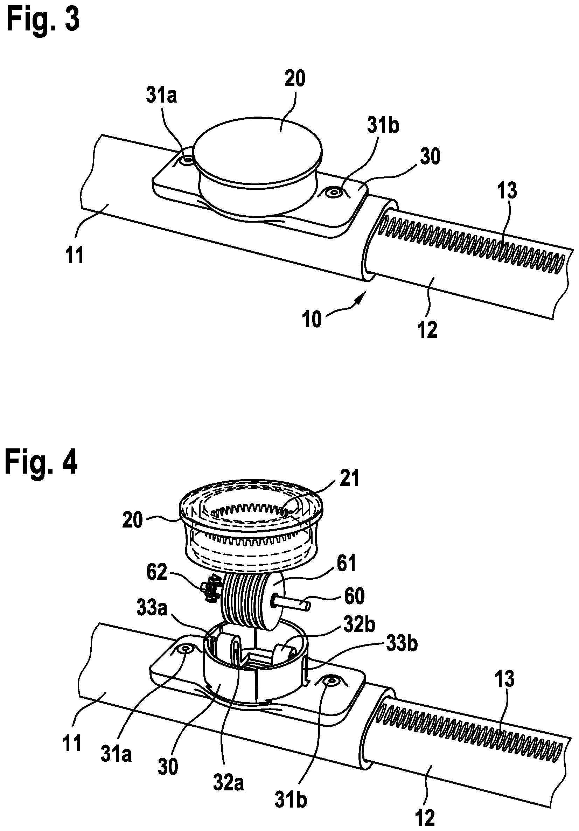

Das Aufnahmeelement 30 ist vorliegend aus einem Kunststoff ausgeführt. Wie in Fig. 3 gezeigt ist, ist es mittels zweier Schrauben 31 a, 31b an der äußeren Schiene 11 befestigt. Der Drehknopf 20 ist auf dem Aufnahmeelement 30 angeordnet und so mit diesem verbunden, dass er zu dem Aufnahmeelement 30 und damit zu der äußeren Schiene 11 hin gedrückt werden kann und auch von der äußeren Schiene 11 weggezogen werden kann, ohne dabei von dem Aufnahmeelement 30 gelöst werden zu können. Zugleich ist er frei drehbar.

Wie in Fig. 4 gezeigt wird, weist das Aufnahmeelement 30 einen hohlzylinderförmigen Bereich auf, in dem erste Führungen 32a, 32b angeordnet sind. Diese weisen jeweils orthogonal zu der äußeren Schiene 11 verlaufende Spalte auf. Zweite Führungen 33a, 33b sind als orthogonal zu der äußeren Schiene 11 verlaufende Spalte ausgeführt, die durch die Außenwand des Aufnahmeelements 30 hindurchgehen. Eine Achse 60 ist durch die Spalte aller Führungen 32a, 32b, 33a, 33b geführt, so dass sie mit ihren beiden Enden über die Außenwand des Aufnahmeelements 30 hinausragt. Zwischen den ersten Führungen 32a, 32b ist ein Schneckenzahnrad 61 auf der Achse 60 angeordnet. Zwischen einer der ersten Führungen 32a und einer der zweiten Führungen 33a ist ein Kegelzahnrad 62 auf der Achse 60 angeordnet. Der Drehknopf 20 weist in seinem Inneren einen umlaufenden Zahnkranz 21 auf, der mit dem Kegelzahnrad 62 eingreift. Gemeinsam mit dem Aufnahmeelement 30 umschließt er das Schneckenzahnrad 61 und das Kegelzahnrad 62.

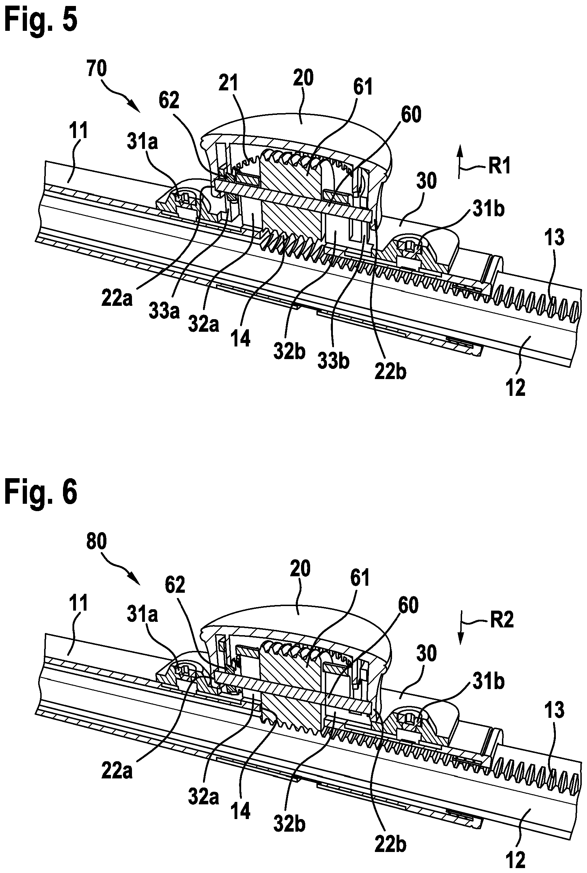

Fig. 5 zeigt den Drehknopf 20 in einer ersten Position 70, in die er gebracht werden kann, indem der Drehknopf 20 in eine erste Richtung R1 gezogen wird. Der Drehknopf 20 weist an seiner Innenseite Eingriffe 22a, 22b auf, in welche die Achse 60 mit ihren über das Aufnahmeelement 30 hinausstehenden Enden eingreift. Auf diese Weise wird die Achse 60 und somit auch das Schneckenzahnrad 61 bei der Bewegung des Drehknopfes 20 in die Richtung R1 von der äußeren Schiene 11 wegbewegt. Über die Achse 60 und die Führungen 32a, 32b, 33a, 33b ist der Drehknopf 20 so mit der Aufnahme 30 verbunden, dass die Bewegung in die Richtung R1 durch ein Anschlagen beendet wird und der Drehknopf nicht von der Aufnahme 30 gelöst werden kann.

Die äußere Schiene 11 weist unterhalb der Aufnahme 30 auf ihrer der Aufnahme 30 zugewandten Seite eine Ausnehmung 14 auf. Diese ist unterhalb des Schneckenzahnrades 61 ausgebildet. Die innere Schiene 12 ist in der äußeren Schiene 11 so geführt, dass ihre Verzahnung 13 dem Schneckenzahnrad 61 zugewandt ist. Wird der Drehknopf 20 durch Drücken in die Richtung R2 in seine zweite Position 80 bewegt, so bewegt er dabei die Achse 60 so lange in Richtung der inneren Schiene 12 bis das Schneckenzahnrad 61 in die Verzahnung 13 der inneren Schiene 12 eingreift.

In der ersten Position 70 ist die innere Schiene 12 frei in der äußeren Schiene 11 bewegbar, so dass die Teleskopschiene 10 an die Beinlänge eines Patienten angepasst werden kann. Sobald der Drehknopf 20 in seine zweite Position 80 gebracht wird, ist ein weiteres Teleskopieren der Teleskopschiene 10 aufgrund der Selbsthemmung zwischen dem Schneckenzahnrad 61 und der Verzahnung 13 nur noch durch ein Drehen des Drehknopfes 20 möglich. Dessen Drehbewegung wird über den Zahnkranz 21, das Kegelzahnrad 62, die Achse 60, das Schneckenzahnrad 61 und schließlich die Verzahnung 13 auf die innere Schiene 12 übertragen. Sobald diese in die für die Versorgung eines Knochenbruches erforderliche Position gebracht wurde, wird das Drehen des Drehknopfes 20 beendet. Er wird in der zweiten Position 80 belassen, um durch den Eingriff zwischen dem Schneckenzahnrad 61 und der Außenverzahnung 13 zu verhindern, dass sich die innere Schiene 12 relativ zur äußeren Schiene 11 verschiebt. Soll die Streckvorrichtung hingegen vom Bein des Patienten entfernt werden, so kann die erreichte Streckung leicht durch ein Herausziehen des Drehknopfes 20 aufgehoben werden.

## Patentansprüche

1. Streckvorrichtung zur Versorgung von Knochenbrüchen, aufweisend
- eine Teleskopschiene (10), weiche eine äußere Schiene (11) und eine in diese einschiebbare innere Schiene (12) aufweist, **dadurch gekennzeichnet, dass** die innere Schiene (12) eine entlang ihrer Längsachse verlaufende Verzahnung (13) aufweist,
- einen Drehknopf (20), der an der äußeren Schiene (11) angeordnet ist und der durch Ziehen von der äußeren Schiene weg (R1) und durch Drücken in Richtung der äußeren Schiene (R2) zwischen einer ersten Position (70) und einer zweiten Position (80) bewegt werden kann, und
- ein Schneckenzahnrad (61), das eingerichtet ist, um durch Drehung des Drehknopfes (20) gedreht zu werden, und das so angeordnet ist, dass es in der ersten Position (70) des Drehknopfes (20) nicht in die Verzahnung (13) eingreift und in der zweiten Position (80) des Drehknopfes (20) in die Verzahnung (13) eingreift.

2. Streckvorrichtung nach Anspruch 1, **dadurch gekennzeichnet, dass** die äußere Schiene (11) eine Ausnehmung (14) aufweist und auf der Ausnehmung (14) ein Aufnahmeelement (30) angeordnet ist, aufweisend
- eine Achse (60), auf der das Schneckenzahnrad (61) angeordnet ist, und
- Führungen (32a, 32b, 33a, 33b), in denen die Achse (60) orthogonal zur äußeren Schiene (11) beweglich angeordnet ist.

3. Streckvorrichtung nach Anspruch 2, **dadurch gekennzeichnet, dass** auf der Achse (60) ein Kegelzahnrad (62) angeordnet ist, und dass der Drehknopf (20) einen innenliegenden Zahnkranz (21) aufweist, welcher mit dem Kegelzahnrad (62) eingreift.

4. Streckvorrichtung nach Anspruch 2 oder 3, **dadurch gekennzeichnet, dass** die Achse (60) in den Drehknopf (20) eingreift.

5. Streckvorrichtung nach einem der Ansprüche 1 bis 4, **dadurch gekennzeichnet, dass** der Drehknopf an dem der inneren Schiene (12) zugewandten Ende der äußeren Schiene (11) angeordnet ist.

6. Streckvorrichtung nach einem der Ansprüche 1 bis 5, **dadurch gekennzeichnet, dass** die Teleskopschiene (10) Gurtbefestigungselemente (41, 42, 43, 44) aufweist, wobei der Drehknopf (20) auf einer von einem Gurtbefestigungselement (42) abgewandten Seite der Teleskopschiene (10) angeordnet ist.

7. Streckvorrichtung nach Anspruch 6, **dadurch gekennzeichnet, dass** die innere Schiene (12) nur ein Gurtbefestigungselement (41) aufweist, welches an dem der äußeren Schiene abgewandten Ende der inneren Schiene (12) angeordnet ist.

8. Streckvorrichtung nach einem der Ansprüche 1 bis 7, **dadurch gekennzeichnet, dass** die Teleskopschiene (10) ein Beckengurtbefestigungselement (44) aufweist, das mit der äußeren Schiene (11) verbunden ist.

## Claims

1. Extension device for treating bone fractures, having
- a telescope rail (10), which has an outer rail (11) and an inner rail (12) that can be shifted into it, **characterised in that** the inner rail (12) has teeth (13) running along its longitudinal axis,
- a rotatory knob (20) which is arranged on the outer rail (11) and which can be moved between a first position (70) and a second position (80) by pulling away from the outer rail (R1) and by pressing in the direction of the outer rail (R2), and
- a worm (61) which is set up to be rotated by the rotation of the rotatory knob (20), and which is arranged in such a way that it does not engage in the teeth (13) in the first position (70) of the rotatory knob (20) and engages in the teeth (13) in the second position (80) of the rotatory knob (20).

2. Extension device according to claim 1, **characterised in that** the outer rail (11) has a recess (14), and a seating element (30) is arranged on the recess (14), having
- an axis (60) on which the worm (61) is arranged, and
- guides (32a, 32b, 33a, 33b), in which the axis (60) is moveably arranged orthogonally to the outer rail (11).

3. Extension device according to claim 2, **characterised in that** a bevel gearwheel (62) is arranged on the axis (60), and the rotary knob (20) has an internal gear rim (21), which engages with the bevel gearwheel (62).

4. Extension device according to claim 2 or 3, **characterised in that** the axis (60) engages in the rotary knob (20).

5. Extension device according to one of claims 1 to 4, **characterised in that** the rotary knob is arranged on the end of the outer rail (11) facing towards the inner rail (12).

6. Extension device according to one claims 1 to 5, **characterised in that** the telescopic rail (10) has belt fastening elements (41, 42, 43, 44), wherein the rotary knob (20) is arranged on a side of the telescopic rail (10) facing away from a belt fastening element (42).

7. Extension device according to claim 6, **characterised in that** the inner rail (12) has only one belt fastening element (41), which is arranged on the end of the inner rail (12) facing away from the outer rail.

8. Extension device according to one of claims 1 to 7, **characterised in that** the telescopic rail (10) has a pelvic belt fastening element (44), which is connected to the outer rail (11).

## Revendications

1. Dispositif de traction pour le soin de fractures osseuses, présentant :
- un rail télescopique (10) présentant un rail extérieur (11) et un rail intérieur (12) insérable dans celui-ci, **caractérisé en ce que** le rail intérieur (12) possède une denture (13) le long de son axe longitudinal,
- un bouton rotatif (20) disposé sur le rail extérieur (11) et mobile entre une première position (70) et une deuxième position (80) selon qu'il est éloigné du rail extérieur en étant tiré (R1) ou rapproché du rail extérieur en étant poussé (R2), et
- une vis sans fin (61) conçue pour tourner sous l'effet de la rotation du bouton rotatif (20) et agencée pour être désengrenée de ladite denture (13) lorsque le bouton rotatif (20) se trouve dans la première position (70) et pour être engrenée dans ladite denture (13) lorsque le bouton rotatif (20) se trouve dans la deuxième position (80).

2. Dispositif de traction selon la revendication 1, **caractérisé en ce que** le rail extérieur (11) présente une exclusion (14) et **en ce qu'**un élément de logement (30) est agencé sur l'évidement (14) et présente
- un axe (60) sur lequel est agencée la vis sans fin (61), et
- des dispositifs de guidage (32a, 32b, 33a, 33b) dans lesquels l'axe (60) est disposé mobile perpendiculairement au rail extérieur (11).

3. Dispositif de traction selon la revendication 2, **caractérisé en ce qu'**une roue conique (62) est agencée sur l'axe (60), et **en ce que** le bouton rotatif (20) présente une couronne dentée (21) interne (21) qui s'engrène avec la roue conique (62).

4. Dispositif de traction selon la revendication 2 ou 3, **caractérisé en ce que** l'axe (60) pénètre dans le bouton rotatif (20).

5. Dispositif de traction selon l'une des revendications 1 à 4, **caractérisé en ce que** le bouton rotatif est agencé sur l'extrémité du rail extérieur (11) qui est tournée vers le rail intérieur (12).

6. Dispositif de traction selon l'une des revendications 1 à 5, **caractérisé en ce que** le rail télescopique (10) présente des éléments de fixation de ceinture (41, 42, 43, 44), ledit bouton rotatif (20) étant agencé sur une face du rail télescopique (10) qui est opposée à un élément de fixation de ceinture (42).

7. Dispositif de traction selon la revendication 6, **caractérisé en ce que** le rail intérieur (12) ne présente qu'un élément de fixation de ceinture (41), lequel est agencé sur l'extrémité du rail intérieur (12) qui est éloignée du rail extérieur.

8. Dispositif de traction selon l'une des revendications 1 à 7, **caractérisé en ce que** le rail télescopique (10) présente un élément de fixation de ceinture de pelvis (44) qui est relié au rail extérieur (11).
